(19) **Europäisches Patentamt**

**European Patent Office**

**Office européen des brevets**

(11) **EP 1 133 962 B1**

(12) **EUROPEAN PATENT SPECIFICATION**

(45) Date of publication and mention
of the grant of the patent:
**29.06.2005 Bulletin 2005/26**

(51) Int Cl.⁷: **A61F 13/15**

(21) Application number: **01302012.8**

(22) Date of filing: **06.03.2001**

(54) **Porous sheet, absorbent article using porous sheet and manufacturing method thereof**

Poröses Blatt, saugfähiger Artikel mit porösem Blatt und Methode zu dessen Herstellung

Feuille poreuse, article absorbant en utilisant la feuille poreuse et la méthode de fabrication de celle

(84) Designated Contracting States:
**DE FR GB NL SE**

(30) Priority: **13.03.2000 JP 2000068287**

(43) Date of publication of application:
**19.09.2001 Bulletin 2001/38**

(73) Proprietor: **Uni-Charm Corporation**
**Shikokuchuo-shi, Ehime-ken (JP)**

(72) Inventors:
• **Mizutani, Satoshi, c/o Technical Center**
**Mitoyo-gun, Kagawa-ken 769-1602 (JP)**
• **Tamura, Tatsuya, c/o Technical Center**
**Mitoyo-gun, Kagawa-ken 769-1602 (JP)**
• **Noda, Yuki, c/o Technical Center**
**Mitoyo-gun, Kagawa-ken 769-1602 (JP)**

(74) Representative: **Fitchett, Stuart Paul**
**Saunders & Dolleymore**
**European Patent Attorneys**
**9 Rickmansworth Road**
**Watford WD18 0JU (GB)**

(56) References cited:
**EP-A- 0 792 629          WO-A-97/02133**

• **PATENT ABSTRACTS OF JAPAN vol. 1996, no.
05, 31 May 1996 (1996-05-31) & JP 08 012792 A
(DAIO PAPER CORP), 16 January 1996
(1996-01-16)**
• **DATABASE WPI Derwent Publications Ltd.,
London, GB; AN 1996-112798 XP002170937 & JP
08 012792 A (DAIO PAPER CORP), 16 January
1996 (1996-01-16)**

**Description**

BACKGROUND OF THE INVENTION

Field of the Invention

The present invention relates to a porous sheet having an uneven surface and a plurality of through holes formed therein, an absorbent article using the porous sheet as a surface sheet or an exterior sheet, and a manufacturing method of the porous sheet.

Description of the Related Art

In absorbent article of the prior art, such as sanitary napkin or disposable diaper, there has been used a sheet formed with an uneven surface (e.g., corrugated surface) as a surface sheet covering the liquid-receiving side of an absorbent layer. In this case, in order to permit blood, urine or the like to pass through it to the absorbent layer, and in order to prevent the blood, urine or the like absorbed in the absorbent layer from flowing back to the wearer's skin, the surface sheet is formed of a hydrophobic non-woven fabric or resin film which is formed with a number of through holes.
The hydrophobic non-woven fabric or resin film is also used for an exterior sheet appearing on an outer surface of the disposable diaper. Especially, when the resin film is used, it is typical to form the through holes in order to provide good air permeability and whereby to prevent a portion of human body where the diaper is applied, from being stuffy. Furthermore, employing a sheet deformed into a corrugated configuration as the exterior sheet, soft feeling may be provided for better appearance.
Conventionally, when a sheet having a corrugated configuration or the like and having a plurality of through holes is formed, the step of shaping the sheet by means of a mold which is provided with an uneven surface is performed separately from the step of forming the plurality of through holes in the sheet.
However, by separately performing the step of shaping the sheet to have an uneven surface and the step of forming the plurality of through holes, the following problems are encountered.
At first, when a plurality of through holes are formed after shaping the sheet to have an uneven surface (e. g., corrugated surface), the unevenness formed in the sheet is possibly crushed during a pin press process, which is typically performed in the through hole forming step, to thereby decrease the top-bottom height of the unevenness in the sheet after formation of the through holes. As a result, cushioning properties of the sheet may be degraded or the appearance may not provide soft feeling.
On the other hand, it is also possible that a number of through holes are firstly formed by pin pressing a flat sheet or by stretching a flat resin film containing filler, and then the flat sheet thus having the through holes is clamped by molds to have an uneven surface. In this case, however, a part of or all of the through holes are crushed by the molds for forming the unevenness to lower the open area ratio.
WO 97/02133 describes corrugated laminates of two or more layers of material and having density differentials created in at least one of the layers of the laminate.
JP 08012792 described the production of an embossed microporous film by pressing a flexible plastic film containing a finely powdered filler between an embossing roll and a counter roll while keeping the film strained. As a result, the film is not only embossed, but also comes to have many micropores as a result of stretching.

SUMMARY OF THE INVENTION

The present invention has an object to provide a porous sheet having a sufficient top-bottom height in unevenness without causing crushing of through holes for achieving both of high cushioning properties and sufficient high open area ratio, and an absorbent article using the porous sheet.
Another object of the present invention is to provide a manufacturing method of a porous sheet which can perform formation of an uneven surface and formation of through holes simultaneously.
According to a first aspect of the present invention, there is provided a porous sheet having an uneven surface and a plurality of through holes formed therein, comprising a sheet including a higher density region and a lower density region, one of which is interspersed in another, wherein the sheet is elongated to form the uneven surface simultaneously with the through holes at the boundary portions between the higher density region and the lower density region.
The porous sheet according to the first aspect of the invention is elongated (or stretched) to have a predetermined uneven surface shape as a finished product. Upon elongation of the sheet, moreover, the through holes are formed due to the density difference between the higher density region and the lower density region. Since the through holes are formed utilizing elongation of the sheet, a great number of through holes can be formed while maintaining the unevenness in sufficient height. Also, the open area ratio can be maintained high.

For example, the sheet may be a non-woven fabric, in which a plurality of higher density regions are formed to be interspersed in the rest portion as the lower density region, so that the through holes are formed at the boundary portions between the higher density regions and the rest portion to be interspersed corresponding to the higher density regions.

In the above, it may be possible that the non-woven fabric includes thermoplastic resin fibers, and that the thermoplastic resin fibers are thermally fused only in the higher density regions or more firmly in the higher density regions than in the rest portion.

When the sheet is formed of the non-woven fabric, it is preferred that a density ratio of the lower density region to the higher density region in the non-woven fabric is greater than or equal to 1% and smaller than or equal to 70% for facilitating formation of the through holes.

For example, it is possible that the uneven surface is of a corrugated configuration, and that the through holes are elongated in a direction along which corrugations are repeated (i.e., in a direction along which the sheet is elongated upon formation of the corrugated configuration).

Alternatively, concavity and convexity may be repeated in at least two directions on the sheet surface.

According to a second aspect of the present invention, there is provided an absorbent article having a surface sheet provided on a liquid-receiving side of an absorbent layer, or having an exterior sheet provided on an opposite side of a liquid-receiving side of an absorbent layer. The surface sheet or the exterior sheet is formed of the porous sheet as set forth above.

According to a third aspect of the present invention, there is provided a method for manufacturing a porous sheet having an uneven surface and a plurality of through holes formed therein, comprising the steps of:

> providing a blank sheet having a higher density region and a lower density region, one of which is interspersed in another; and
> clamping the sheet between forming dies having uneven surfaces mutually meshing with each other to form the sheet with an uneven surface while being elongated along the uneven surfaces of the forming dies, whereupon the sheet is formed with a plurality of through holes at the boundary portions between the higher density region and the lower density region due to the elongation at formation of the uneven surface.

In the porous sheet manufacturing method, formation of the unevenness and formation of the plurality of through holes can be performed simultaneously in the same process step.

For example, the sheet may be a non-woven fabric, in which a plurality of higher density regions are formed to be interspersed in the rest portion as the lower density region. In this case, it is possible that the non-woven fabric includes thermoplastic resin fibers, and that the thermoplastic resin fibers are thermally fused only in the higher density regions or more firmly in the higher density regions than in the rest portion.

In the case where the forming dies are designed for forming the non-woven fabric with a corrugated configuration, corrugations of which are repeated and arranged in a machine direction of the non-woven fabric, a breaking elongation of the non-woven fabric in the machine direction is preferably set to be greater than or equal to 50% and smaller than or equal to 100% (more preferably, greater than or equal to 70% and smaller than or equal to 100%) of an elongation percentage of the non-woven fabric in the machine direction upon formation of the corrugations by the forming dies.

In the case where the forming dies are designed for forming the non-woven fabric with a corrugated configuration, corrugations of which are repeated and arranged in a cross direction of the non-woven fabric, on the other hand, a density ratio of the lower density region to the higher density region in the non-woven fabric is preferably greater than or equal to 1% and smaller than or equal to 55%, and a breaking elongation of the non-woven fabric in the cross direction is preferably set to be greater than or equal to 15% and smaller than or equal to 100% (more preferably, greater than or equal to 20% and smaller than or equal to 70%) of an elongation percentage of the non-woven fabric in the cross direction upon formation of the corrugations by the forming dies.

BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:

> Fig. 1A is a perspective view of a sheet having higher density regions and a lower density region;
> Fig. 1B is a perspective view of a porous sheet after processing;
> Fig. 2 is a perspective view showing a processing step employing shaping rolls;

Fig. 3 is a sectional view showing an uneven shaping surface of the shaping roll;

Fig. 4 is a section of a non-woven fabric formed with unevenness;

Fig. 5A is an enlarged plan view showing a through hole when the non-woven fabric is elongated in MD;

Fig. 5B is an enlarged plan view showing a through hole when the non-woven fabric is elongated in CD;

Fig. 6 is a diagrammatic illustration showing a relationship between a unit width load and elongation percentage;

Fig. 7 is a perspective view of a sanitary napkin as an absorbent article using a porous sheet; and

Fig. 8 is a perspective view of a disposable diaper as an absorbent article using a porous sheet.

DESCRIPTION OF THE PREFERRED EMBODIMENT

The present invention will be discussed hereinafter in detail in terms of the preferred embodiment of the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structure is not shown in detail in order to avoid unnecessary obscurity of the present invention.

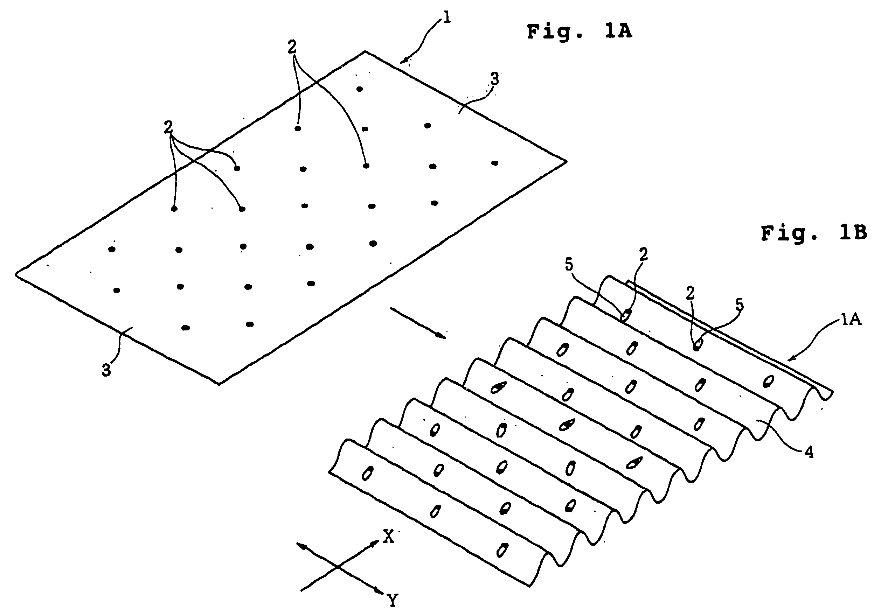

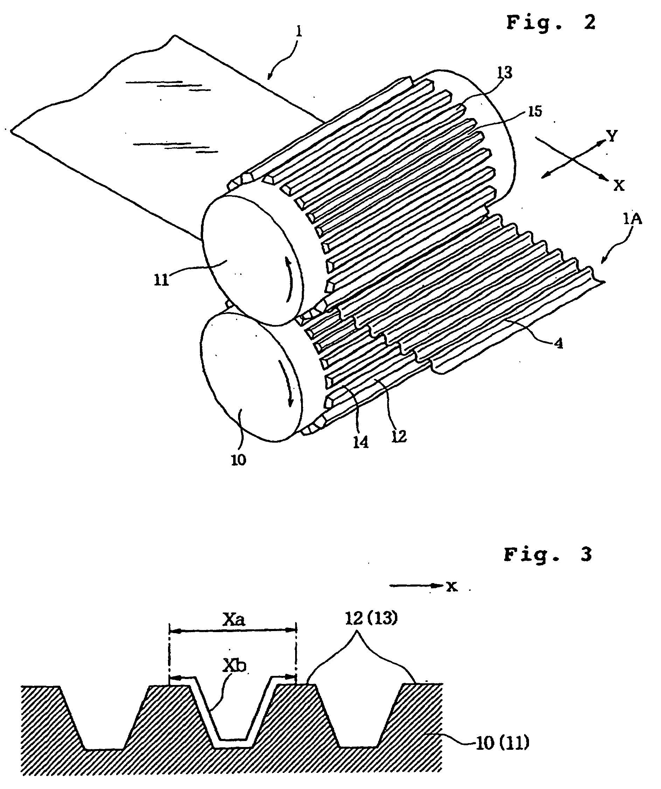

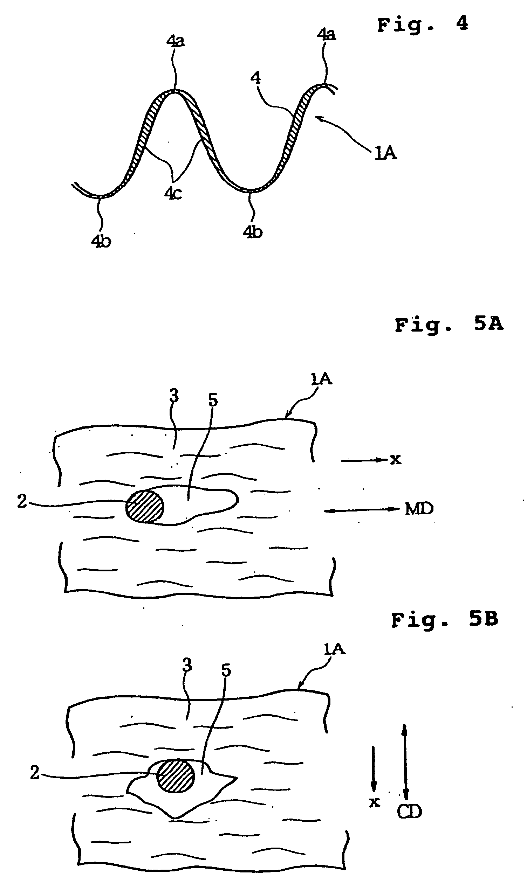

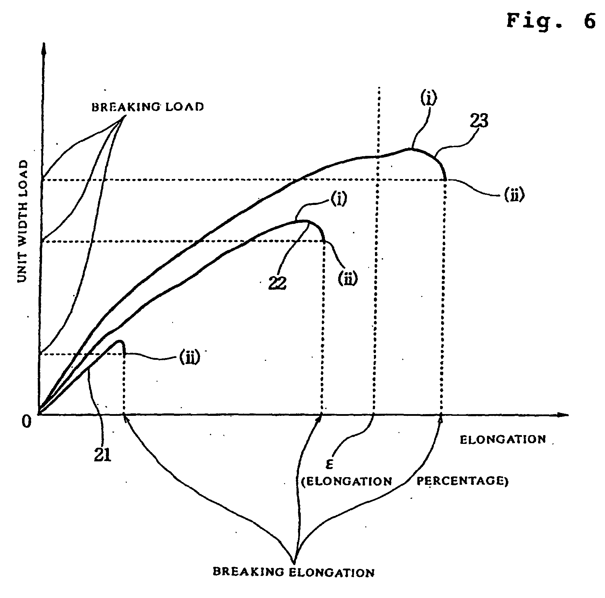

Fig. 1A is a perspective view of a sheet having higher density regions and a lower density region; Fig. 1B is a perspective view of a porous sheet after processing; Fig. 2 is a perspective view showing a processing step employing shaping rolls; Fig. 3 is a sectional view showing an uneven shaping surface of the shaping roll; Fig. 4 is a section of a non-woven fabric formed with unevenness; Fig. 5A is an enlarged plan view showing a through hole (or opening) when the non-woven fabric is elongated in MD; Fig. 5B is an enlarged plan view showing a through hole when the non-woven fabric is elongated in CD; and Fig. 6 is a diagrammatic illustration showing a relationship between a unit width load and elongation percentage.

Fig. 1A shows a sheet 1 before shaping process (before processing). As shown as black dots, higher density regions 2 having a higher density than that of the rest portion, are interspersed in the sheet 1. The rest portion other than the higher density regions 2 is referred to as a lower density region 3 having a lower density than that of the higher density regions 2. A density ratio derived from (density of lower density region 3)/(density of higher density region 2) is preferably in a range of greater than or equal to 1% to less than or equal to 70%. An area ratio of the higher density region 2 in the entire surface of the sheet is preferably in a range of greater than or equal to 5% to smaller than or equal to 20%

In the invention, the sheet 1 is formed of a non-woven fabric.

The higher density regions 2 can be formed by locally applying pressure under heat (i.e., by heat embossing). In this case, it is preferred that the non-woven fabric is formed only of thermoplastic resin fibers (fusible fibers) or is formed of the thermoplastic resin fibers together with other fibers. In such a non-woven fabric containing the thermoplastic resin fibers, the higher density regions 2 can be formed by locally heating and pressurizing the non-woven fabric to thereby thermally fuse the thermoplastic resin fibers. In an alternative, the higher density regions 2 may be formed such that the thermoplastic resin fibers are thermally fused both in the higher density regions 2 and the lower density region 3 but more firmly in the higher density regions 2. The non-woven fabric containing the thermoplastic resin fibers may be exemplified by a point-bonded non-woven fabric using short fibers or a spun-bonded non-woven fabric using long fibers. Also, the higher density regions 2 may be formed by locally heating and pressurizing a through-air non-woven fabric, a melt-blown non-woven fabric or the like.

As the thermoplastic resin fibers forming the non-woven fabric, use can be made of fusible synthetic fibers such as those of PE (polyethylene), PP (polypropylene) or PET (polyethylene terephthalate) or core-sheath type or side-by-side type composite synthetic fibers such as those of PE/PP or PE/PET.

In an alternative, the higher density regions 2 may be formed by locally heating and pressurizing a non-woven fabric not containing thermoplastic resin fibers such as a spun-laced non-woven fabric formed of rayon or the like. In this case, the constituent fibers are not thermally fused but compressed in the higher density regions 2. In another alternative, the higher density regions 2 may be formed by applying resin such as an adhesive to be interspersed on any one of the various non-woven fabrics thus far described.

Here, when the higher density region 2 is formed by locally heating and pressurizing the non-woven fabric, the density ratio can be derived by basis weight (this may be referred to as "Metsuke") of the non-woven fabric and thickness of the higher density region 2, and the basis weight and thickness of the lower density region 3. Smaller density ratio represents higher density in the higher density region 2 relative to the density of the lower density region 3.

Fig. 2 shows shaping rolls 10 and 11 to be used for forming a porous sheet 1A having an uneven surface from the sheet 1.

The shaping rolls 10 and 11 have mutually the same construction and have surfaces formed with a plurality of shaping ribs (linear emboss or stripe emboss) 12 and 13 extending in parallel to axes of the rolls 10 and 11 (Y direction). The shaping ribs 12 and 13 are arranged with a given pitch in the circumferential direction. The shaping rolls 10 and 11 are heated. When the sheet 1 is formed of the non-woven fabric of thermoplastic resin fibers, surface temperature of the shaping ribs 12 and 13 is set at a temperature lower by 10°C to 50°C than the melting point of the thermoplastic resin forming the non-woven fabric.

The lower shaping roll 10 and the upper shaping roll 11 are combined to mesh the shaping ribs 12 and 13 with each other. Namely, the shaping ribs 12 of the lower shaping roll 10 may penetrate into grooves 15 defined between adjacent shaping ribs 13 of the upper shaping roll 11, and the shaping ribs 13 of the upper shaping roll 11 may penetrate into grooves 14 defined between adjacent shaping ribs 12 of the lower shaping roll 10.

When the sheet 1 is supplied between the rolls 10 and 11 in the condition where the shaping roll 10 and the shaping roll 11 are in rotation, the sheet 1 is clamped by the shaping ribs 12 and the shaping ribs 13 to obtain the porous sheet 1A formed with the uneven surface 4. In the porous sheet 1A, ridge portions and valley portions of the uneven surface 4 are repeated at a given pitch in a feeding direction of the sheet by the shaping rolls 10 and 11 (X direction) and are extended individually in a width direction of the porous sheet 1A (Y direction). Namely, the uneven surface 4 of the porous sheet 1A is of a corrugated or wavy configuration (stripe emboss pattern). Fig. 4 is an enlarged section of the porous sheet 1A formed of the non-woven fabric.

Fig. 3 is the section showing a shape of the shaping rib 12 of the shaping roll 10. As can be seen from Fig. 3, the shaping rib 12 has trapezoidal cross-section. The shaping rib 13 of the shaping roll 11 also has the same cross-sectional shape as shown in Fig. 3.

The sheet 1 having density difference is clamped between the shaping roll 10 and the shaping roll 11. Then, the sheet 1 is clamped between the shaping rib 12 and the shaping rib 13 and is elongated (or stretched) in the feeding direction (X direction). In Fig. 3, an arrangement pitch of the shaping ribs 12 (or 13) in the circumferential direction is indicated at Xa, and a length along the uneven surface of the shaping roll 10 (or 11) in the pitch Xa is indicated at Xb. When the sheet 1 is clamped by the shaping ribs 12 and 13, therefore, the sheet 1 is elongated from the length Xa to the length Xb in the X direction.

Accordingly, elongation percentage of the sheet 1 in the X direction can be expressed by

$$\{(Xb - Xa)/Xa\} \times 100 \ (\%)$$

In practice, the sheet 1 may not be elongated from the length Xa to the length Xb occasionally due to slippage between the sheet 1 and the shaping ribs 12 and 13. However, in the following disclosure, the elongation percentage of the sheet 1 by the shaping rolls is referred to the value derived from the foregoing expression on the basis of Xa and Xb derived from the sectional shape of the shaping ribs 12 and 13.

By setting a ratio of breaking elongation in the feeding direction (X direction) of the sheet 1 to the foregoing elongation percentage within a predetermined range and by setting a density ratio of the lower density region 3 to the higher density region 2 within a predetermined range, when the sheet 1 is elongated, through holes 5 are formed mainly in the lower density region 3 due to rupture caused at the boundary between the higher density region 2 and the lower density region 3, as shown in Fig. 1B. The through holes 5 are elongated in the elongating direction of the sheet 1 (X direction). In the porous sheet 1A after formation, the through holes 5 are distributed corresponding to all of or most of the higher density regions 2.

Fig. 6 illustrates elongation-load characteristic curves of three kinds of sheets (samples 21, 22 and 23) having the higher density regions 2. These samples were elongated in one direction. In Fig. 6, a vertical axis represents a tensile load per a unit width (unit is inch or cm), and horizontal axis represents an elongation (expressed by elongation percentage). Assuming that a length of the sample before elongation is L and a length of the sample after elongation by a predetermined unit load is $(L + \Delta L)$, the elongation percentage is expressed by:

$$(\Delta L/L) \times 100 \ (\%)$$

In the individual characteristic curves, (i) indicates a yield point where tensile force of the sheet becomes maximum; (ii) indicates a breaking point where rapture of the sheet is started and subsequently rapture is expanded. The elongation percentage at the breaking point (ii) represents breaking elongation, and the load on the unit width at the breaking point (ii) represents breaking load.

Here, it is assumed that the elongation percentage by the shaping rolls 10 and 11 of Fig. 2 for forming (or shaping) the uneven surface 4 on the sheet 1 is set at $\varepsilon$. At this time, since the breaking elongation (or the elongation percentage at the breaking point) of the sample 21 is too small with respect to the elongation percentage $\varepsilon$, the sample 21 breaks into pieces as fed by the shaping rolls 10 and 11. In the sample 23, on the other hand, the elongating percentages at the yield point (i) and at the breaking point (ii) are sufficiently higher than the foregoing elongation percentage $\varepsilon$. Therefore, the elongation percentage $\varepsilon$ is in a range where the sample 23 can elastically stretch. As a result, when processed by the shaping rolls 10 and 11, the sample 23 can be formed with the corrugated uneven surface 4 but not with the through holes 5.

In contrast to this, the breaking elongation of the sample 22 is lower than but sufficiently close to the elongation percentage ε. Accordingly, when the sample 22 is processed by the shaping rolls 10 and 11 to form the uneven surface 4, rapture is caused at the boundary between the higher density region 2 and the lower density region 3 and subsequently elongated, so that the through holes 5 are distributed corresponding to the higher density regions 2.

It should be noted that although the preferred breaking elongation of the sheet 1 relative to the elongation percentage ε may vary depending upon property of the sheet 1, the density difference between the higher density region 2 and the lower density region 3 and so on, the through hole 5 can be formed by appropriately setting the breaking elongation within a range of at least 20% to at most 100 % the elongation percentage ε.

Fig. 4 is a section of the corrugated uneven surface 4 in which the sheet 1 is formed of a non-woven fabric. When the non-woven fabric is clamped by the shaping rib 12 of the shaping roll 10 and the shaping rib 13 of the shaping roll 11 and thus is subject to a stripe embossing process, the fiber density becomes higher in the ridge portions 4a and the valley portions 4b than in the side wall portions 4c. As a result, when load is applied from the above, the side wall portions 4c of a lower fiber density are allowed to expand sidewardly to provide soft feeling as contacting with a skin of human body. On the other hand, when the load is removed, the original condition is restored elastically. In short, the corrugated uneven surface 4 can exhibit elastic cushioning properties.

Figs. 5A and 5B show the higher density region 2 and the through hole 5 in an enlarged scale. When the sheet 1 is formed of a non-woven fabric, the liquid, as has been given to the lower density region 3, is drawn to the higher density region 2 to flow into the through hole 5 through the circumferential portion of the higher density region 2. Thus, the liquid can easily pass through the sheet. Accordingly, the porous sheet 1A becomes optimal for use as a surface sheet of an absorbent article, such as sanitary napkin, disposable diaper and so on.

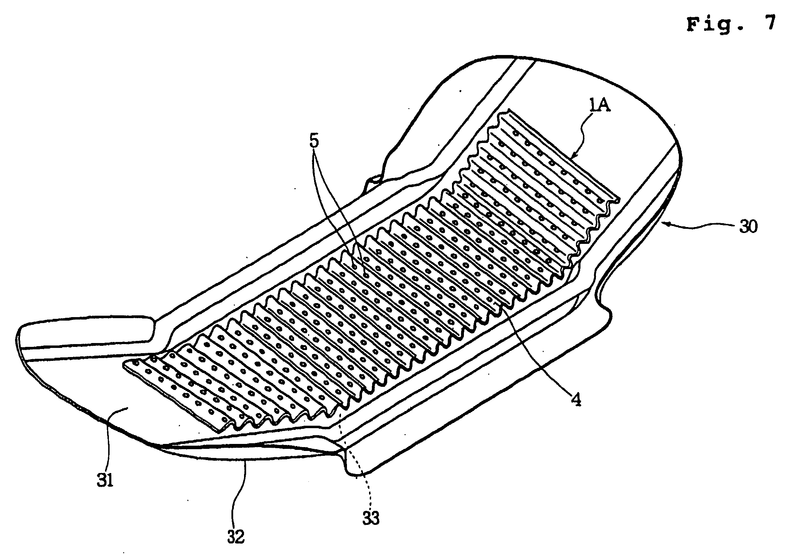

Fig. 7 is a perspective view showing a sanitary napkin 30 as one example of an absorbent article using the porous sheet 1A.

In the sanitary napkin 30, an absorbent core (or absorbent layer) 33 is sandwiched between a liquid permeable top sheet 31 and a liquid impermeable back sheet 32. On the top sheet 31, there is provided a surface sheet which is formed of the porous sheet 1A. In an alternative, the porous sheet 1A may be directly disposed on the liquid-receiving surface of the absorbent core 33, in place of the top sheet 31. When the porous sheet 1A is used as the surface sheet, agreeable feeling may be provided as contacting with skin of the wearer, and menstrual blood can be absorbed in the absorbent core 33 through the through holes 5.

The porous sheet 1A is formed of a non-woven fabric. It is preferably formed of hydrophobic fibers or fibers subjected to a hydrophobic treatment. When the porous sheet 1A is hydrophobic, the menstrual blood absorbed in the absorbent core 33 hardly returns toward the wearer to avoid sticky feeling of the liquid-receiving surface.

In the embodiment shown in Fig. 7, the uneven surface 4 of the porous sheet 1A is of corrugations repeated in the longitudinal direction of the sanitary napkin 30, the ridge portions and valley portions of which extend in the width direction of the sanitary napkin 30. Accordingly, the menstrual blood given to the porous sheet 1A will pass through the through holes 5 to be absorbed in the absorbent core 33 while being prevented from spreading in the longitudinal direction of the sanitary napkin 30. Thus, leakage of menstrual blood in the longitudinal direction can be prevented effectively.

It may also be possible to provide the porous sheet 1A in such a manner that the uneven surface 4 is of corrugations repeated in the width direction and the ridge portions and valley portions thereof extend in the longitudinal direction.

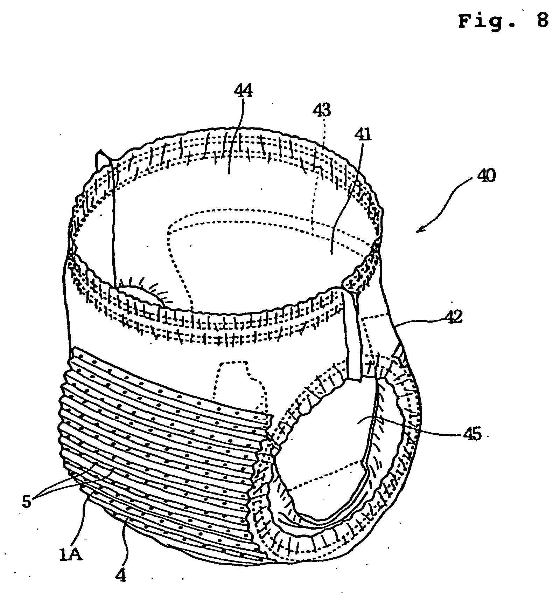

Fig. 8 is a perspective view of an example where the porous sheet 1A is used in a disposable diaper 40.

In the disposable diaper 40, an absorbent core 43 is sandwiched between a liquid permeable top sheet 41 and a liquid impermeable back sheet 42. The disposable diaper 40 is a so-called "pants type" disposable diaper formed with a waist opening 44 and a pair of leg openings 45 and 45. However, the porous sheet according to the present invention is also applicable for an open type disposable diaper.

As an exterior sheet of the disposable diaper 40, the porous sheet 1A is provided on the surface of the back sheet 42. The porous sheet 1A may also be used in place of the back sheet 42 covering the outer surface of the absorbent core 43. The porous sheet 1A used as the exterior sheet is formed of a hydrophobic non-woven fabric to have the through holes 5. Since the exterior sheet has the through holes 5, good air permeability can be provided. On the other hand, since the exterior sheet has the uneven surface 4, soft appearance can be provided.

It should be noted that, while the uneven surface of the porous sheet 1A is of corrugations repeated in one direction in the shown embodiment, it is also possible to provide unevenness repeated in at least two directions (e.g., in both of X and Y directions). For instance, the porous sheet may be embossed to have a plurality of circular or quadrangular projecting portions repeated regularly or irregularly on its surface.

[Examples 1]

Using the shaping roll 10 and the shaping roll 11 having the uneven surfaces of the stripe emboss pattern shown in Fig. 2, the following treatment of non-woven fabrics of the example 1 and comparative examples was performed. In the example 1, the non-woven fabric was fed orienting the machine direction thereof in the feeding direction (X direction) of the shaping rolls 10 and 11 to perform formation of the corrugated uneven surface 4, and in conjunction therewith, formation of the through holes 5.

The shaping ribs 12 and 13 of the shaping rolls 10 and 11 used in the foregoing process had trapezoidal cross-section as shown in Fig. 3. A surface temperature of the shaping rolls 10 and 11 was set at 100°C. By pressurization of the shaping rolls 10 and 11, a pressure of 27.46 kPa was applied to perform formation.

In the shaping rolls, Xa was 1.36 mm, and Xb was 3.0 mm. Therefore, the elongation percentage was

$$\{(Xb - Xa)/Xa\} \times 100 = 120.6 \ (\%)$$

(Example 1)

A spun-bonded non-woven fabric formed of core-sheath type composite synthetic fibers (long fibers) of PE/PP, having basis weight of 30 g/m$^2$ was used. The higher density regions 2 were formed by pin embossing to be interspersed as shown in Fig. 1A. The elongation-load characteristics shown in Fig. 6 was that breaking load in MD was 35.28N per one inch width, breaking elongation in MD was 97% and density ratio was 64%.

(Comparative Example 1)

A spun-bonded non-woven fabric formed of core-sheath type composite synthetic fibers (long fibers) of PE/PP, having basis weight of 30 g/m$^2$ was used. Breaking elongation in MD was 150% and density ratio was 60%. Since the breaking elongation in MD was high, its breaking load in MD could not be measured.

(Comparative Example 2)

A spun-bonded non-woven fabric formed of fibers (long fibers) of PP, having basis weight of 22 g/m$^2$ was used. Breaking load in MD was 28.42N per one inch width, breaking elongation in MD was 41% and density ratio was 52%.

(Comparative Example 3)

A spun-bonded non-woven fabric formed of fibers (long fibers) of PP, having basis weight of 20 g/m$^2$ was used. Breaking load in MD was 17.84N per one inch width, breaking elongation in MD was 16% and density ratio was 50%.

(Comparative Example 4)

A point-bonded non-woven fabric formed of core-sheath type composite synthetic fibers (short fibers) of PE/PP, having basis weight of 20 g/m$^2$ was used. Breaking load in MD was 10.78N per one inch width, breaking elongation in MD was 20% and density ratio was 60%.

(Evaluation)

Concerning formability of unevenness, one, in which a corrugated uneven surface was formed, was evaluated as acceptable or good and indicated by "○", and one, in which a corrugated uneven surface was not formed and breakage or rapture was caused in the portions other than the through holes 5 of the sheet, was evaluated as not acceptable or no good and indicated by "X".

Concerning formability of through holes, one, in which the through holes 5 were formed in greater than or equal to 60% of the higher density regions 2, was evaluated as acceptable or good and indicated by "○", and one, in which the through holes 5 were formed less than 60% of the higher density regions 2 or the through holes 5 were never formed, was evaluated as not acceptable or no good and indicated by "X".

The following table 1 shows the results of evaluation.

TABLE 1

| Sample | Breaking Elongation (%) | Breaking Elongation/ Elongation Percentage (%) | Density Ratio (%) | Unevenness Formability | Through Hole Formability |
|---|---|---|---|---|---|
| Ex. 1 | 97 | 80.4 | 64 | ○ | ○ |
| Comp. 1 | 150 | 124.4 | 60 | ○ | x |
| Comp. 2 | 41 | 34.0 | 52 | x | ○ |
| Comp. 3 | 16 | 13.3 | 50 | x | ○ |
| Comp. 4 | 20 | 16.6 | 60 | x | ○ |

In the comparative example 1, since the elongation percentage by the shaping rolls 10 and 11 was sufficiently greater than the breaking elongation of the sheet, breakage or rapture was not caused at the boundary between the higher density region 2 and the lower density region 3 upon formation of unevenness. Therefore, the through hole 5 was not formed.

In the comparative examples 2 and 3, since the ratios of the breaking elongation (elongation percentage at break) to the elongation percentage (120.6%) were as low as less than 50%, the sheets were broken without formation of the corrugated uneven surface upon formation by the shaping rolls 10 and 11.

In contrast to this, in the example 1, the ratio of the breaking elongation to the elongation percentage (120.6%) was appropriate, as 80.4% to demonstrate superior unevenness formability and through hole formability.

As seen from the above, when the non-woven fabric is elongated (stretched) in the machine direction (MD) as shown in Fig. 5A to form the corrugated uneven surface and at the same time, to form the through holes, the ratio of the breaking elongation to the elongation percentage exerts a more significant influence than the density ratio. In order to form the unevenness in MD and simultaneously form the through holes, it is preferred that the ratio of the breaking elongation to the elongation percentage is in a range greater than or equal to 50% and smaller than or equal to 100%, further preferably in a range greater than or equal to 70% and smaller than or equal to 100%.

[Examples 2]

Using the shaping roll 10 and the shaping roll 11 having the uneven surfaces of the stripe emboss pattern shown in Fig. 2, the following treatment of non-woven fabrics of the examples and comparative examples was performed. In the example 2, the non-woven fabric was fed orienting the cross direction (CD) thereof in the feeding direction (X direction) of the shaping rolls 10 and 11 to perform formation of the corrugated uneven surface 4, and in conjunction therewith, formation of the through holes 5.

The shaping ribs 12 and 13 of the shaping rolls 10 and 11 used in the foregoing process had trapezoidal cross-section as shown in Fig. 3. A surface temperature of the shaping rolls 10 and 11 was set at 100°C. By pressurization of the shaping rolls 10 and 11, a pressure of 27.46 kPa was applied to perform formation.

In the shaping rolls, Xa was 1.36 mm, and Xb was 3.0 mm. Therefore, the elongation percentage was 120.6 (%).

(Example 2a)

A spun-bonded non-woven fabric formed of PP fibers (long fibers), having basis weight of 22 g/m$^2$ was used. The higher density regions 2 were formed by pin embossing to be interspersed as shown in Fig. 1A. The elongation-load characteristics shown in Fig. 6 was that breaking load in CD was 7.06N per one inch width, breaking elongation in CD was 53% and density ratio was 52%.

(Example 2b)

A spun-bonded non-woven fabric formed of PP fibers (long fibers), having basis weight of 20 g/m$^2$ was used. Breaking load in CD was 5.49N per one inch width, and breaking elongation in CD was 27% and density ratio was 50%.

(Comparative Example 2a)

A point-bonded non-woven fabric formed of core-sheath type composite synthetic fibers (short fibers) of PE/PP, having basis weight of 20 g/m$^2$ was used. Breaking load in CD was 1.47N per one inch width, and breaking elongation in CD was 55% and density ratio was 60%.

(Comparative Example 2b)

A spun-bonded non-woven fabric formed of core-sheath type composite synthetic fibers (long fibers) of PE/PP, having basis weight of 30 g/m$^2$ was used. Breaking load in CD was 12.35N per one inch width, and breaking elongation in CD was 110% and density ratio was 64%.

(Evaluation)

Concerning formability of unevenness, one, in which a corrugated uneven surface was formed, was evaluated as acceptable or good and indicated by "○", and one, in which a corrugated uneven surface was not formed and breakage or rapture was caused in the portions other than the through holes 5 of the sheet, was evaluated as not acceptable or no good and indicated by "X". One, in which uniformly corrugated uneven surface was not formed as a whole, was evaluated as indeterminable and indicated by "Δ".

Concerning formability of through holes, one, in which the through holes 5 were formed in greater than or equal to 60% of the higher density regions 2, was evaluated as acceptable or good and indicated by "○", and one, in which the through holes 5 were formed less than 60% of the higher density regions 2 or the through holes 5 were never formed, was evaluated as not acceptable or no good and indicated by "X". One, in which through holes were formed but were interspersed randomly in the lower density region 3 not at the boundary between the higher density region 2 and the lower density region 3, was evaluated as indeterminable and indicated by "Δ".

The following table 2 shows the results of evaluation.

TABLE 2

| Sample | Breaking Elongation (%) | Breaking Elongation/ Elongation Percentage(%) | Density Ratio (%) | Unevenness Formability | Through Hole Formability |
|---|---|---|---|---|---|
| Ex. 2a | 53 | 43.9 | 52 | ○ | ○ |
| Ex. 2b | 27 | 22.4 | 50 | ○ | ○ |
| Comp. 2a | 55 | 45.6 | 60 | Δ | Δ |
| Comp. 2b | 110 | 91.2 | 64 | ○ | x |

In the comparative example 2a, since the density ratio was too high, namely, the density of the higher density region 2 was too low, the through holes were not formed at the boundary between the higher density region 2 and the lower density region 3 and rather formed were cracks slashing the fibers in the lower density region 3, and thus the shape of the uneven surface was not formed uniformly. The comparative example 2b had an appropriate ratio of breaking elongation to the elongation percentage but since the density ratio was too high, namely the density of the higher density region 2 was too low, no apparent through hole was formed.

When formation of unevenness and formation of through holes are performed by stretching the non-woven fabric in CD, as in the above, the load for stretching acts in CD (or the direction perpendicular to MD along which the individual fibers extend). Therefore, the through holes 5 are formed by separating fibers, as located at the boundary between the higher density region 2 and the lower density region 3, from one another in CD. For the function set forth above, the through hole formability is significantly influenced by density ratio. Furthermore, it becomes necessary to appropriately set the ratio of the breaking elongation to the elongation percentage. Accordingly, as seen from the table 2, in the elongation in CD, it is preferred that the density ratio is greater than or equal to 1% and less than or equal to 55%, and the ratio of the breaking elongation to the elongation percentage is greater than or equal to 15% and less than or equal to 100%, and more preferably greater than or equal to 20% and less than or equal to 70%.

Here, it should be noted that the shaping rolls may have shaping ribs each extending continuously in the circumferential direction and arranged at an given pitch in the roll axis direction for elongating the non-woven fabric in CD. In this case, the non-woven fabric can be fed orienting its MD in the feeding direction of the shaping rolls so that the productivity is improved.

As set forth above, according to the present invention, the porous sheet superior in the unevenness formability and high in the open area ratio can be obtained. On the other hand, formation of unevenness and formation of through holes in the sheet can be performed in the same process step simultaneously.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

**Claims**

1. A porous sheet (1) having an uneven surface and a plurality of through holes (5) formed therein, comprising
   a sheet including a higher density region (2) and a lower density region (3), one of which is interspersed in another,
   wherein the sheet (1) is elongated to form the uneven surface (4) simultaneously with the through holes (5) at the boundary portions between the higher density region (2) and the lower density region (3),
   wherein the sheet (1) is a non-woven fabric, in which a plurality of higher density regions (2) are formed to be interspersed in the rest or remaining portion of the lower density region (3), so that the through holes (5) are formed at the boundary portions between the higher density regions (2) and the rest or remaining portion of the lower density region and are interspersed corresponding to the higher density regions (3).

2. The porous sheet as set forth in Claim 1, wherein the non-woven fabric includes thermoplastic resin fibers, and the thermoplastic resin fibers are thermally fused only in the higher density regions (2) or more firmly in the higher density regions than in the rest portion (3).

3. The porous sheet as set forth in Claim 1, wherein a density ratio of the lower density region to the higher density region in the non-woven fabric is greater than or equal to 1% and smaller than or equal to 70%.

4. The porous sheet as set forth in Claim 1, wherein the uneven surface is of a corrugated configuration, and the through holes (5) are elongated in a direction along which corrugations are repeated.

5. The porous sheet as set forth in Claim 1, wherein concavity and convexity are repeated in at least two directions on the sheet surface.

6. An absorbent article (30) having a surface sheet (31) provided on a liquid-receiving side of an absorbent layer (33), the surface sheet (31) being formed of a porous sheet (1A) having an uneven surface and a plurality of through holes (5) formed therein, the porous sheet (1A) comprising
   a sheet including a higher density region and a lower density region, one of which is interspersed in another,
   wherein the sheet (1A) is elongated to form the uneven surface (4) simultaneously with the through holes (5) at the boundary portions between the higher density region and the lower density region,
   wherein the sheet is a non-woven fabric in which a plurality of higher density regions are formed to be interspersed in the rest or remaining portion of the lower density region, so that the through holes (5) are formed at the boundary portions between the higher density regions and the rest or remaining portion of the lower density region and are interspersed corresponding to the higher density regions.

7. An absorbent article (40) having an exterior sheet provided on an opposite sheet of a liquid-receiving side of an absorbent layer, the exterior sheet (1A) being formed of a porous sheet having an uneven surface (4) and a plurality of through holes (5) formed therein, the porous sheet comprising
   a sheet including a higher density region and a lower density region, one of which is interspersed in another,
   wherein the sheet is elongated to form the uneven surface simultaneously with the through holes (5) at the boundary portions between the higher density region and the lower density region,
   wherein the sheet is a non-woven fabric in which a plurality of higher density regions are formed to be interspersed in the rest or remaining portion of the lower density region, so that the through holes (5) are formed at the boundary portions between the higher density regions and the rest or remaining portion of the lower density region and are interspersed corresponding to the higher density regions.

8. A method for manufacturing a porous sheet having an uneven surface and a plurality of through holes formed therein, comprising the steps of:

   providing a blank sheet having a higher density region and a lower density region, one of which is interspersed in another; and
   clamping the sheet between forming dies having uneven surfaces mutually meshing with each other to form the sheet with an uneven surface while being elongated along the uneven surfaces of the forming dies, whereupon the sheet is formed with a plurality of through holes at the boundary portions between the higher density region and the lower density region due to the elongation at formation of the uneven surface, wherein the sheet is a non-woven fabric, in which a plurality of higher density regions are formed to be interspersed in the rest or remaining portion of the lower density region.

9. The porous sheet manufacturing method as set forth in Claim 8, wherein the non-woven fabric includes thermoplastic resin fibers, and the thermoplastic resin fibers are thermally fused only in the higher density regions or more firmly in the higher density regions than in the rest portion.

10. The porous sheet manufacturing method as set forth in Claim 8, wherein the forming dies are designed for forming the non-woven fabric with a corrugated configuration, corrugations of which are repeated and arranged in a machine direction of the non-woven fabric, and
    wherein a breaking elongation of the non-woven fabric in the machine direction is set to be greater than or equal to 50% and smaller than or equal to 100% of an elongation percentage of the non-woven fabric in the machine direction upon formation of the corrugations by the forming dies.

11. The porous sheet manufacturing method as set forth in Claim 8 , wherein the forming dies are designed for forming the non-woven fabric with a corrugated configuration, corrugations of which are repeated and arranged in a cross direction of the non-woven fabric, and
    wherein a density ratio of the lower density region to the higher density region in the non-woven fabric is greater than or equal to 1% and smaller than or equal to 55%, and a breaking elongation of the non-woven fabric in the cross direction is set to be greater than or equal to 15% and smaller than or equal to 100% of an elongation percentage of the non-woven fabric in the cross direction upon formation of the corrugations by the forming dies.


**Patentansprüche**

1. Poröses Blatt (1) mit einer unebenen Oberfläche und mehreren darin gebildeten Durchgangslöchern (5), mit
   einem Blatt mit einem Bereich höherer Dichte (2) und einem Bereich niedrigerer Dichte (3), von denen der eine den anderen durchsetzt,
   wobei das Blatt (1) gedehnt wird, um die unebene Oberfläche (4) gleichzeitig mit den Durchgangslöchern (5) an den Randabschnitten zwischen dem Bereich höherer Dichte (2) und dem Bereich niedrigerer Dichte (3) zu bilden,
   wobei das Blatt (1) in einem Vliesstoff besteht, in dem mehrere Bereiche höherer Dichte (2) so ausgebildet sind, daß sie den Rest oder verbleibenden Abschnitt des Bereichs niedrigerer Dichte (3) durchsetzen, so daß die Durchgangslöcher (5) an den Randabschnitten zwischen den Bereichen höherer Dichte (2) und dem Rest oder verbleibenden Abschnitt des Bereichs niedrigerer Dichte gebildet werden und entsprechend den Bereichen höherer Dichte (3) eingestreut sind.

2. Poröses Blatt nach Anspruch 1, wobei der Vliesstoff thermoplastische Harzfasern aufweist, die nur in den Bereichen höherer Dichte (2) oder stärker in den Bereichen höherer Dichte als in dem Restabschnitt (3) thermisch verschmolzen sind.

3. Poröses Blatt nach Anspruch 1, wobei das Dichteverhältnis des Bereichs niedrigerer Dichte zu dem Bereich höherer Dichte in dem Vliesstoff mindestens 1% und höchstens 70% beträgt.

4. Poröses Blatt nach Anspruch 1, wobei die unebene Oberfläche eine Wellenkonfiguration aufweist, und die Durchgangslöcher (5) in Richtung der sich wiederholenden Wellen gedehnt sind.

5. Poröses Blatt nach Anspruch 1, wobei sich Konkavität und Konvexität in wenigstens zwei Richtungen der Blattoberfläche wiederholen.

**6.** Saugfähiger Artikel (30) mit einer Oberflächenlage (31), die an einer flüssigkeitsaufnehmenden Seite einer saugfähigen Lage (33) vorgesehen ist, wobei

die Oberflächenlage (31) aus einem porösen Blatt (1A) mit einer unebenen Oberfläche und mehreren darin gebildeten Durchgangslöchern (5) gebildet ist,

das poröse Blatt (1A) ein Blatt mit einem Bereich höherer Dichte und einem Bereich niedrigerer Dichte aufweist, von denen der eine den anderen durchsetzt,

das Blatt (1A) gedehnt wird, um die unebene Oberfläche (4) gleichzeitig mit den Durchgangslöchern (5) an den Randabschnitten zwischen dem Bereich höherer Dichte und dem Bereich niedrigerer Dichte zu bilden, und

das Blatt in einem Vliesstoff besteht, in dem mehrere Bereiche höherer Dichte dazu ausgebildet sind, den Rest oder verbleibenden Abschnitt des Bereichs niedrigerer Dichte zu durchsetzen, so daß die Durchgangslöcher (5) an den Randabschnitten zwischen den Bereichen höherer Dichte und dem Rest oder verbleibenden Abschnitt des Bereichs niedrigerer Dichte gebildet werden und entsprechend den Bereichen höherer Dichte eingestreut sind.

**7.** Saugfähiger Artikel (40) mit einer Außenlage, die an einer der flüssigkeitsaufnehmenden Seite entgegengesetzten Lage vorgesehen ist, wobei

die Außenlage (1A) aus einem porösen Blatt mit einer unebenen Oberfläche (4) und mehreren darin gebildeten Durchgangslöchern (5) gebildet ist,

das poröse Blatt ein Blatt mit einem Bereich höherer Dichte und einem Bereich niedrigerer Dichte aufweist, von denen der eine den anderen durchsetzt,

das Blatt gedehnt wird, um die unebene Oberfläche gleichzeitig mit den Durchgangslöchern (5) an den Randabschnitten zwischen dem Bereich höherer Dichte und dem Bereich niedrigerer Dichte zu bilden, und

das Blatt in einem Vliesstoff besteht, in dem mehrere Bereiche höherer Dichte dazu ausgebildet sind, den Rest oder verbleibenden Abschnitt des Bereichs niedrigerer Dichte zu durchsetzen, so daß die Durchgangslöcher (5) an den Randabschnitten zwischen den Bereichen höherer Dichte und dem Rest oder verbleibenden Abschnitt des Bereichs niedrigerer Dichte gebildet werden und entsprechend den Bereichen höherer Dichte eingestreut sind.

**8.** Verfahren zum Herstellen eines porösen Blattes mit einer unebenen Oberfläche und mehreren darin gebildeten Durchgangslöchern, wobei

ein unbehandeltes Blatt mit einem Bereich höherer Dichte und einem Bereich niedrigerer Dichte, die einander durchsetzen, bereitgestellt wird, und

das Blatt zwischen Formwalzen geklemmt wird, die unebene, ineinandergreifende Oberflächen aufweisen, um dem Blatt eine unebene Oberfläche zu geben, während es längs der unebenen Oberflächen der Formwalzen gedehnt wird, wobei das Blatt durch die Dehnung bei der Bildung der unebenen Oberfläche mit mehreren Durchgangslöchern an den Randbereichen zwischen dem Bereich höherer Dichte und dem Bereich niedrigerer Dichte gebildet wird, wobei das Blatt in einem Vliesstoff besteht, in dem mehrere Bereiche höherer Dichte so gebildet sind, daß sie den Rest oder verbleibenden Abschnitt des Bereichs niedrigerer Dichte durchsetzen.

**9.** Herstellungsverfahren für ein poröses Blatt nach Anspruch 8, wobei der Vliesstoff thermoplastische Harzfasem aufweist, die nur in den Bereichen höherer Dichte oder stärker in den Bereichen höherer Dichte als in dem Restabschnitt thermisch verschmolzen sind.

**10.** Herstellungsverfahren für ein poröses Blatt nach Anspruch 8, wobei die Formwalzen dazu ausgelegt sind, dem Vliesstoff eine Wellenkonfiguration zu geben, deren Wellen sich wiederholen und in einer Maschinenrichtung des Vliesstoffs angeordnet sind, und

wobei die Bruchdehnung des Vliesstoffs in der Maschinenrichtung auf mindestens 50% und höchstens 100% des Dehnungsprozentsatzes des Vliesstoffs in der Maschinenrichtung nach der Wellenbildung durch die Formwalzen eingestellt ist.

**11.** Herstellungsverfahren für ein poröses Blatt nach Anspruch 8, wobei die Formwalzen dazu ausgelegt sind, dem Vliesstoff eine Wellenkonfiguration zu geben, deren Wellen sich wiederholen und in einer Querrichtung des Vliesstoffs angeordnet sind, und

wobei das Dichteverhältnis des Bereichs niedrigerer Dichte zu dem Bereich höherer Dichte in dem Vliesstoff mindestens 1% und höchstens 55% beträgt, und die Bruchdehnung des Vliesstoffs in der Querrichtung auf mindestens 15% und höchstens 100% des Dehnungsprozentsatzes des Vliesstoffs in der Querrichtung nach Bildung der Wellen durch die Formwalzen eingestellt ist.

**Revendications**

1.  Feuille poreuse (1) ayant une surface irrégulière et une pluralité de trous de passage (5) ménagés dans celle-ci; comprenant

    une feuille comprenant une région (2) à densité élevée et une région (3) à faible densité, l'une d'elles étant parsemée dans l'autre,

    dans laquelle la feuille (1) est allongée de manière à constituer la surface irrégulière (4) en même temps que les trous de passage (5) aux parties limites entre la région (2) à densité élevée et la région (3) à faible densité,

    dans laquelle la feuille (1) est une étoffe non tissée où une pluralité de régions (2) à densité élevée sont constituées pour être parsemées dans le reste ou la partie restante de la région (3) à faible densité, de manière que les trous de passage (5) soient ménagés aux parties limites entre les régions (2) à densité élevée et le reste ou la partie restante de la région à faible densité et soient parsemés en correspondance avec les régions (2) à densité élevée.

2.  Feuille poreuse selon la revendication 1, dans laquelle l'étoffe non tissée comprend des fibres de résine thermoplastique, et les fibres de résine thermoplastique ne sont fondues thermiquement que dans les régions (2) à densité élevée, ou de manière plus poussée dans les régions à densité élevée que dans la partie restante (3).

3.  Feuille poreuse selon la revendication 1, dans laquelle un rapport de densité entre la région à faible densité et la région à densité élevée dans l'étoffe non tissée est supérieur ou égal à 1 % et inférieur ou égal à 70 %.

4.  Feuille poreuse selon la revendication 1, dans laquelle la surface irrégulière est à configuration ondulée, et les trous de passage (5) sont allongés dans une direction selon laquelle les ondulations sont répétées.

5.  Feuille poreuse selon la revendication 1, dans laquelle une concavité et une convexité sont répétées dans au moins deux directions sur la surface de la feuille.

6.  Article absorbant (30) comportant une feuille de surface (31) placée sur le côté recevant du liquide d'une couche absorbante (33), la feuille de surface (31) étant constituée d'une feuille poreuse (1A) ayant une surface irrégulière et une pluralité de trous de passage (5) ménagés dans celle-ci, la feuille poreuse (1A) comprenant

    une feuille comprenant une région à densité élevée et une région à faible densité, l'une d'elles étant parsemée dans l'autre,

    dans lequel la feuille (1A) est allongée de manière à constituer la surface irrégulière (4) en même temps que les trous de passage (5) aux parties limites entre la région à densité élevée et la région à faible densité,

    dans lequel la feuille est une étoffe non tissée, où plusieurs régions à densité élevée sont constituées pour être parsemées dans le reste ou la partie restante de la région à faible densité, de manière que les trous de passage (5) soient ménagés aux parties limites entre les régions à densité élevée et le reste ou la partie restante de la région à faible densité et soient parsemés soient parsemés en correspondance avec les régions à densité élevée.

7.  Article absorbant (40) comportant une feuille extérieure placée sur une feuille opposée d'un côté recevant du liquide d'une couche absorbante, la feuille extérieure (1A) étant constituée d'une feuille poreuse ayant une surface irrégulière (4) et une pluralité de trous de passage ménagés dans celle-ci, la feuille poreuse comprenant

    une feuille comprenant une région à densité élevée et une région à faible densité, l'une d'elles étant parsemée dans l'autre,

    dans lequel la feuille est allongée de manière à constituer la surface irrégulière en même temps que les trous de passage (5) aux parties limites entre la région à densité élevée et la région à faible densité,

    dans lequel la feuille est une étoffe non tissée, où des régions à densité élevée d'une pluralité sont constituées pour être parsemées dans le reste ou la partie restante de la région à faible densité, de manière que les trous de passage (5) soient ménagés aux parties limites entre les régions à densité élevée et le reste ou la partie restante de la région à faible densité et soient parsemées en correspondance avec les régions à densité élevée.

8.  Procédé de fabrication d'une feuille poreuse ayant une surface irrégulière et des trous de passage ménagés dans celle-ci, comprenant les étapes consistant à :

    fournir une feuille brute comportant une région à densité élevée et une région à faible densité, l'une d'elles étant parsemée dans l'autre ; et
    bloquer la feuille entre des matrices de formage ayant des surfaces irrégulières se mettant mutuellement en prise l'une avec l'autre afin de former la feuille avec une surface irrégulière tout en l'allongeant selon les surfaces irrégulières des matrices de formage, après quoi la feuille est constituée avec une pluralité de trous de passage aux parties limites entre la région à densité élevée et la région à faible densité du fait de l'allongement au cours de la formation de la surface irrégulière, dans lequel la feuille est une étoffe non tissée où une pluralité de régions à densité élevée est formée de manière à être parsemée dans le reste ou la partie restante de la région à faible densité.

9.  Procédé de fabrication de feuille poreuse selon la revendication 8, dans lequel l'étoffe non tissée comprend des fibres de résine thermoplastique, et les fibres de résine thermoplastique ne sont fondues thermiquement que dans les régions (2) à densité élevée, ou de manière plus poussée dans les régions à densité élevée que dans la partie restante.

10. Procédé de fabrication de feuille poreuse selon la revendication 8, dans lequel les matrices de formage sont conçues pour former l'étoffe non tissée avec une configuration ondulée, dont les ondulations sont répétées et disposées dans une direction de fabrication de l'étoffe non tissée, et

    dans lequel un allongement de rupture de l'étoffe non tissée dans la direction de fabrication est fixé de manière à être supérieur ou égal à 50 % et inférieur ou égal à 100 % d'un pourcentage d'allongement de l'étoffe non tissée dans la direction de fabrication lors de la formation des ondulations par les matrices de formage.

11. Procédé de fabrication de feuille poreuse selon la revendication 8, dans lequel les matrices de formage sont conçues pour former l'étoffe non tissée avec une configuration ondulée, dont les ondulations sont répétées et disposées dans une direction transversale de l'étoffe non tissée, et

    dans lequel un rapport de densité entre la région à faible densité et la région à densité élevée dans l'étoffe non tissée est supérieur ou égal à 1 % et inférieur ou égal à 55 %, et un allongement de rupture de l'étoffe non tissée dans la direction transversale est fixé de manière à être supérieur ou égal à 15 % et inférieur ou égal à 100 % d'un pourcentage d'allongement de l'étoffe non tissée dans la direction transversale lors de la formation des ondulations par les matrices de formage.

Fig. 1A

Fig. 1B

Fig. 2

Fig. 3

Fig. 4

Fig. 5A

Fig. 5B

Fig. 6

Fig. 7

Fig. 8